# EUROPEAN PATENT SPECIFICATION

(11) **EP 3 407 784 B1**
(45) Date of publication and mention of the grant of the patent: **04.03.2020**
(21) Application number: 17707098.4
(22) Date of filing: 27.01.2017
(51) Int. Cl.: A61B 5/0205, A61B 5/00

(54) **VITAL SIGNS MONITOR/MEASUREMENT APPARATUS**
VITALPARAMETERÜBERWACHUNGS-/-MESSVORRICHTUNG
APPAREIL DE SURVEILLANCE/MESURE DES SIGNES VITAUX

(30) Priority: 28.01.2016 GB 201601593; 28.01.2016 GB 201601591
(43) Date of publication of application: 05.12.2018
(73) Proprietor: Metix Limited, London EC2A 1AF (GB)
(72) Inventor: ONTIVEROS, Julio Enrique Guerrero, Glasgow Strathclyde G5 0TG (GB)
(74) Representative: Hutchison, Craig McGregor
(86) International application number: PCT/GB2017/050223
(87) International publication number: WO 2017/130000

(56) References cited:
- CN-A- 104 720 752
- US-A1- 2014 051 939
- US-A1- 2014 275 817
- US-A1- 2015 087 933
- US-A1- 2015 223 705
- US-A1- 2015 366 471

## Description

### Field of the invention

This invention relates to a vital signs monitor, preferably a portable, hand-held, device for monitoring vital signs of a patient, such as pulse, blood pressure and respiration, and a vital signs monitoring system.

### Background to the invention

In medical use, vital signs refers to basic physiological indicators, commonly comprising pulse rate, respiration (breathing rate), temperature and blood pressure. Other signs are sometimes included, such as pulse rate oximetry, cardiac output and invasive blood pressure.

Instruments for monitoring individual vital signs, and certain combinations of vital signs, are well known. However, the majority of these are designed for use in hospitals and other fixed locations. There is a need for a readily-portable, self-contained, handheld device which can monitor multiple vital signs not only in sophisticated hospitals but also in the field, for example in ambulances and in remote unsophisticated medical facilities.

The present inventor has appreciated the shortcomings with known vital signs monitors.

It would also be useful to facilitate vital signs which have been monitored to be aggregated for the purpose of epidemiological studies or quality monitoring purposes.

US patent application US 2015/223705 A1 discloses a vital signs monitor comprising a housing, a power supply and a plurality of measurement modules within the housing. Each module is operable to measure and monitor at least one respective vital sign. The vital signs monitor includes one or more inputs for connection to sensors applied to a patent and arranged to supply sensor signals to respective measurement modules. The monitor also includes a user display and the measurement modules are operable to communicate wirelessly with external sensors.

According to a first aspect of the present invention there is provided a vital signs monitor comprising:
a housing;
a power supply;
a plurality of measurement modules within the housing, each module being operable to measure and monitor at least one respective vital sign;
one or more inputs for connection to sensors applied to a patient and arranged to supply sensor signals to respective measurement modules; and
a user display, wherein the measurement modules are operable to communicate wirelessly with external sensors, the vital signs monitor has near field communication (NFC) transmission and receiving capabilities, such that the monitor may communicate with a further vital signs monitor using NFC technology, the further vital signs monitor comprising:
   a housing;
   a power supply;
   a plurality of measurement modules within the housing, each module being operable to measure and monitor at least one respective vital sign;
   one or more inputs for connection to sensors applied to a patient and arranged to supply sensor signals to respective measurement modules; and
   a user display, and
characterised in that the vital signs monitor includes one or more tracking devices, the one or more tracking devices being operable to signal an alarm when an external sensor moves out of a predetermined range with respect to the vital signs monitor.

The vital signs monitor may also comprise a user input device, the user input device being operable to allow a user to input data, patient details, control parameter, operating parameters, or the like, to the monitor.

The vital signs monitor may be a portable vital signs monitor. The vital signs monitor may be a hand-held monitor. The vital signs monitor may be ergonomically shaped with respect to a user's hand. The vital signs monitor may include one or more apparatus or user attachment devices, the user attachment devices being configured such that the monitor may attachable to an apparatus or a user. The user attachment device may be a strap. The strap may be a hand strap. The strap may be configured such that it may be wrapped around the hand of a user when the user is holding the monitor. The strap may be a wrist strap or a torso strap.

The housing may be of a size suitable to be carried by a user. The housing may be of a size suitable to be carried in one hand by a user.

The housing may have dimensions of less than 200mm x 100mm x 100mm. The housing may have dimensions of less than 170mm x 90mm x 65mm.

The housing may be weatherproof. The housing may be sand proof. The housing may be shock proof. The housing may be submergible. The housing may be sand proof. The housing may have a micro-bacterial coating. The monitor may be weatherproof. The monitor may be sand proof. The monitor may be shock proof. The monitor may be submergible. The monitor may be sand proof. The monitor may have a micro-bacterial coating.

The housing may be made from a plastics material. The housing may be made from injection moulded plastic. The housing may be made from a thermoplastics material. The housing may be made from a metal material. The housing may be made from an alloy material. The housing may be made form a thermoplastic polyester alloy material.

The housing may be flame retardant. The housing may be made from a flame retardant material.

The monitor may be shielded to electromagnetic interference (EMI). The monitor may be EMI-shielded. The monitor may be resistant to ultraviolet (UV) radiation.

The monitor may be capable of operating between temperatures of -30°C to 50 °C. The monitor may be capable of operating at a relative humidity of 15 to 95% (non-condensing).

The power supply is contained within the housing. The power supply may be a self-contained power supply. The power supply may be a rechargeable battery. The power supply may be a rechargeable lithium-ion battery. The power supply may be a mains power supply. The mains power supply may be a medical grade power supply.

The measurement modules may be located within the housing.

The measurement modules are electronic modules.

The measurement modules may be operable to measure at least one additional parameter in addition to the respective vital sign. The measurement modules may be operable to measure two or more vital signs.

The measurement modules are operable to measure and/or monitor vital signs selected from: pulse, blood pressure, temperature, tympanic temperature, electrocardiogram (ECG), respiration, pulse oximetry, cardiac output and capnography. Each measurement module may be operable to measure and/or monitor vital signs selected from: pulse, blood pressure, invasive blood pressure, temperature, electrocardiogram (ECG), respiration, pulse oximetry, cardiac output and capnography.

The measurement modules are sensor modules. The monitor may comprise a blood pressure measurement module, an invasive blood pressure measurement module, an ECG measurement module, a pulse oximetry module, a cardiac output module or one or more combinations of these. The monitor may comprise a blood pressure measurement module, an invasive blood pressure measurement module, an ECG measurement module, a pulse oximetry module, a cardiac output module, or a capnography module, or one or more combinations of these.

The measurement modules may be operable with external sensors. The external sensors may be a blood pressure sensor, an invasive blood pressure sensor, a temperature sensor, an ECG sensor, a respiration sensor, a pulse oximetry sensor, a cardiac output sensor, or a capnography sensor. The monitor may include one or more external sensors. The external sensors may be a blood pressure sensor, an invasive blood pressure sensor, a temperature sensor, an ECG sensor, a respiration sensor, a pulse oximetry sensor, a cardiac output sensor, or a capnography sensor.

The blood pressure sensor may be a non-invasive blood pressure (NIBP) sensor. The blood pressure sensor may be an invasive blood pressure sensor. The blood pressure sensor may be located within an arm cuff, or the like. The blood pressure module may be operable to measure blood pressure at intervals of 10, 15, 30 or 60 minutes. The measurement may have a manual or automatic start/stop function. The blood pressure module may have a measurement time of 30 to 45 seconds (on deflation) and 15 to 30 seconds (on inflation). The blood pressure module may have a measurement range of 20 to 260 mmHg (systolic) and 10 to 220 mmHg (diastolic).

The ECG sensor may include sensors that are attachable to a patient. The sensors may be chest electrodes, paddles, or the like. The ECG module may be operable with 3, 5 or 12 lead cables. The ECG sensor may be operable to measure heart rate in the range 30 to 300 bpm. The ECG module may be operable to measure cardiac pacing. The ECG module may be operable to measure rectilinear, constant width current pulses of 40 ms ± 2 ms at a pacer rate of 30 to 180 bpm. Such measurements may be external transcutaneous.

The ECG module may be operable to provide impedance pneumography. The ECG module may be operable to measure breath rate. The ECG module may be operable to measure breath rate between 2 to 150 breaths per minute. The ECG module may be operable to display the numeric breath rate. The module may be operable to display the impedance waveform. The ECG module may be operable to measure and/or monitor an averaged breath rate. The ECG module may be operable to activate an alarm for low, high and no breath rates.

The pulse oximetry sensor may be a non-invasive sensor. The pulse oximetry sensor may be a non-invasive light transmission sensor. The pulse oximetry sensor may be operable to measure SpO2, pulse rate and perfusion index. The pulse oximetry sensor may be operable to additionally measure total haemoglobin, oxygen content, carboxyhaemoglobin, methaemoglobin, and pleth variability index.

The capnography sensor may be a non-invasive sensor. The capnography sensor may be a non-invasive light transmission sensor or an electromechanical sensor, or an electrochemical sensor. The capnography sensor may be operable to measure end-tidal CO2 (etCO2). The capnography sensor may be operable to provide early indication of evolving respiratory compromise.

Each measurement module may be operable to provide instantaneous data measurements and/or historical data measurements. The data measurements may be presented numerically and/or visually in a plurality of different formats. Each measurement module may perform calculations that may be used as base sensed parameters, early deterioration notifications for individual parameters and different versions of patient early warning score based on multiple parameters. Each measurement module may be operable to predict trends and/or deterioration and provide warning alarms.

The monitor may include one or more alarm devices. The one or more alarm devices being operable to signal an alarm upon measurement of a vital sign having one or more predetermined signals or predetermined values or conditions.

The monitor may be operable to provide a user with information relating to a last known location of the measurement module/sensor module. This information may be presented visually.

The one or more inputs include ports for connection cables. The ports may include cover members. The cover members may be resilient plugs that are at least partially locatable within the ports. The cover members may be configured to mitigate water ingress to the ports.

The monitor is configured such that the one or more sensors communicate wirelessly with each measurement module. The sensor associated with each measurement module is configured to communicate wirelessly therewith. The communication protocol may be Bluetooth, Bluetooth 4.0, Bluetooth 4.1, or the like.

The monitor may also comprise a network module, the network module being operable to control the communication between the measurement module sensors and the measurement modules. The network module may be operable to wirelessly control the communication between the measurement module sensors and the measurement modules.

The network module may also be operable to control the communication between the monitor and/or the measurement modules thereof, with one or more external devices or networks. The network module may be operable to wirelessly control the communication between the monitor and/or the measurement modules thereof, with one or more external devices or networks. The communication may be by cellular (mobile) telephony, or by Wi-Fi over a local area network (LAN), or the like.

The monitor may be configured to be operable with a remote application server. The remote application server may be operable to communicate with other devices, web applications or mobile clients. The remote application server may be operable to communicate with other vital sign monitors. The remote application server may be operable to communicate with one or more other vital sign monitors according to the first aspect of the invention. The remote application server may be configured to provide real-time patient monitoring to store and/or export patient sensing logs (data). The remote application server may be configured to provide global positioning system (GPS) data, or the like, which may be provided in real-time. The remote application server may be configured to provide tracking of the monitor, or multiple monitors. The remote application server may be configured to provide estimated time of arrival (ETA) to care centres, or the like, with push notifications, or the like.

The remote application server may be configured to search within the stored records for users, patients, sensed parameters and/or device events, notifications or alerts, or the like. The search may be performed by requesting particular historic values, patient numbers, targeted search of early warning signs and/or pathological values, or the like. The searches may be performed with text searching and/or speech recognition, or the like.

The remote application server may be configured to interact with and/or be accessed through compatible other platforms (telehealth platforms) and/or other electronic health records.

The monitor may be configured to communicate with the remote application server and other monitors using NFC technology.

The server may be cloud-based.

The remote application server may be able to use data to create a reporting framework. The reporting framework may be configured to store data that corresponds to vital sign monitoring sessions. The data may be presented using standard export formats. The formats may be: comma separated value (csv), MS Excel (xlsx), and PDF. Comma separated value (csv) formats allow for the manipulation of data through commercial off the shelf software. PDF formats enables the data to be presented using proprietary formats that may match the look and feel of a healthcare institution.

The reporting framework may be configured to run in its own web application. The reporting framework may be an independent module to achieve reusability.

The remote application may fully integrate the reporting framework with other patient file systems. This may decrease the navigation steps to get to patient information.

The monitor may also be operable to produce video reporting and data snapshots generation. The monitor may be operable to perform a feasibility study on how useful it would be to make the monitoring data anonymous so that it can be accessed openly throughout the world, most specifically by research centres and universities.

The monitor may have artificial intelligence (AI) algorithms that learn when data is normally requested. This provides the ability to automatically generate data reports based on the patient's condition or the context the patient is in.

The monitor may further comprise an ECG module capable of supplying cardiac pulses. The monitor may further comprise an ECG module capable of supplying cardiac pacing pulses.

The monitor may further comprise an internal memory. The internal memory may be removable from the monitor. The monitor may further comprise two memories, one being selectively removable from the monitor.

The monitor may further comprise an output device for communication with a remote server. The output device may be the network module.

The user display may be located on a side surface of the housing. The user display may be located on one face of the housing.

The user input device may be operable to allow a user to input data to the monitor. The user input device may be operable to allow a user to operate the monitor. The user input device may include a touch screen display.

The vital signs monitor may also comprise a loudspeaker device and/or a microphone.

According to a second aspect of the present invention there is provided a vital signs monitoring system comprising:
two or more vital signs monitors, each vital signs monitor comprising:
   a housing;
   a power supply;
   a plurality of measurement modules within the housing, each module being operable to measure and monitor at least one respective vital sign;
   one or more inputs for connection to sensors applied to a patient and arranged to supply sensor signals to respective measurement modules; and
   a user display; and
a server,
wherein each vital signs monitor is independently operable to provide a local display, and data is transferred between each vital signs monitor and the server, wherein each vital signs monitor has near field communication (NFC) transmission and receiving capabilities, such that each vital signs monitor may communicate with the other vital signs monitor(s) using NFC technology,
the measurement modules are operable to communicate wirelessly with external sensors, and
characterised in that each monitor includes one or more tracking devices, the one or more tracking devices being operable to signal an alarm when an external sensor moves out of a predetermined range with respect to the monitor.

The data may be transferred between each vital signs monitor and the server asynchronously.

The data may be stored in both the respective vital signs monitor and the server.

The server may be a central server.

The server may be operable to aggregate data from a population of individual data sets.

Each measurement module may have a unique identifier and/or location.

Embodiments of the second aspect of the present invention may include one or more features of the first aspect of the present invention or their embodiments.

### Brief description of the drawings

Embodiments of the invention and example apparatus suitable for use with the invention will now be described, by way of example, with reference to the drawings, in which:
Figs. 1a to 1h are views of a vital signs monitor forming one embodiment of the present invention;
Fig. 2 is a schematic block diagram of the monitor of Fig. 1;
Fig. 3 shows an example of a screen display;
Fig. 4 illustrates a networked system;
Figs. 5a and 5b are views of a vital signs measurement apparatus, which is suitable for use with the present invention;
Fig. 6 shows the vital signs measurement apparatus of Figs 5a and 5b in use on a patient, or wearer of the apparatus;
Fig. 7 is a schematic view of a further vital signs measurement apparatus, which is for use with the present invention, in use on a patient, or wearer of the apparatus;
Fig. 8 is a schematic block diagram of the vital signs measurement apparatus of Fig. 5a;
Fig. 9 is a schematic block diagram of the vital signs measurement apparatus of Fig. 7;
Fig. 10 is a schematic block diagram of another example of the vital signs measurement apparatus of Fig. 5a; and
Fig. 11 is a schematic block diagram illustrating the near field communication (NFC) technology of the present invention.

### Description of preferred embodiments

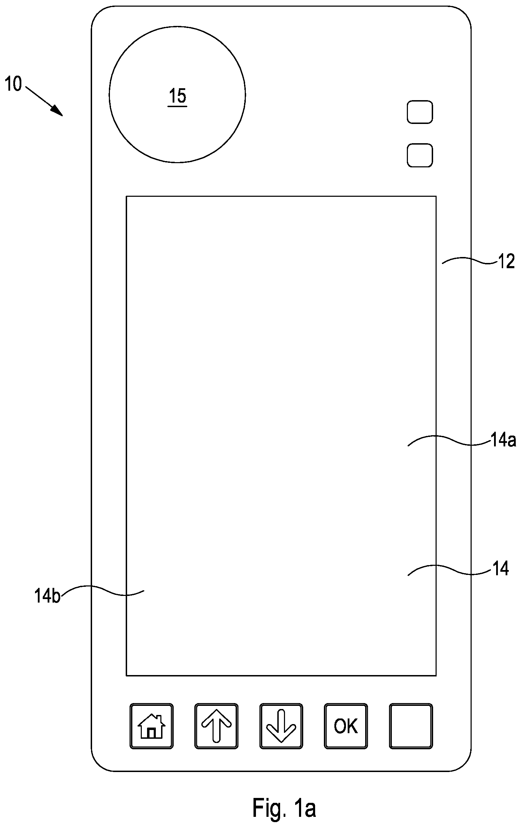

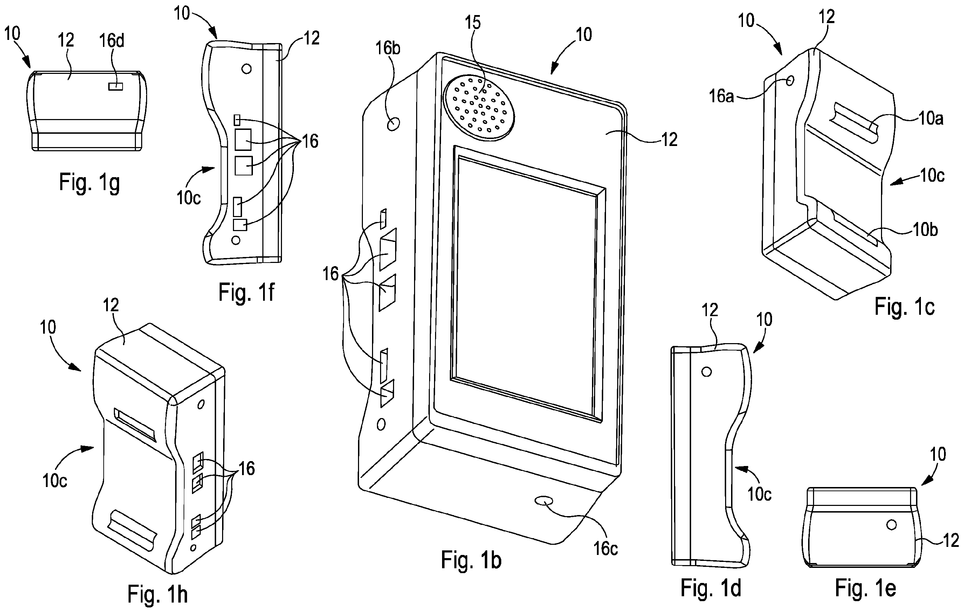

Referring to Figs. 1a to 1h, in which Fig. 1a is a front view of a vital signs monitor 10, Fig. 1b is a perspective front view, Fig. 1c is a perspective rear view, Fig. 1d is a right side view, Fig. 1e is a bottom view, Fig. 1f is a left side view, Fig. 1g is a top view and Fig. 1h is further rear perspective view, a vital signs monitor 10 has a housing 12 including a touch screen 14 and a number of connection ports 16, 16a, 16b, 16c, 16d (an example of one or more inputs). Note that in Figs. 1b to 1h the touch screen 14 and many of the internal components have been omitted for clarity. The connection ports 16, 16a, 16b, 16c, 16d may include cover members (not illustrated). The cover members may be resilient plugs that are at least partially locatable within the ports. The cover members may be configured to mitigate water ingress to the ports. Although the monitor 10 has been illustrated as including two navigation button (UP arrow and DOWN arrow), it should be appreciated that the monitor 10 may include four navigation buttons (UP, DOWN, LEFT and RIGHT arrows). These buttons may be in the membrane.

The housing 12 is designed to be hand held, and typically has dimensions of approximately 170mm x 90mm x 65mm. As best illustrated in Figs. 1c, 1d, 1f and 1h, the monitor 10 is ergonomically shaped with respect to a user's hand, as illustrated generally at 10c. The monitor 10 is also provided with a strap (an example of a user attachment device). The strap, which has been omitted for clarity may be attached to the rear of the monitor via strap attachment points 10a and 10b, as illustrated in Figs. 1c and 1h. The vital signs monitor is therefore portable. The monitor 10 is ruggedised and proof against ingress of water, shock and sand, suitably to IP67 standard; means of achieving such standards are well known. The monitor may also have a micro-bacterial coating.

The housing 12 should be of a size suitable to be carried in one hand, and held in one hand during use. In general terms, this means a size of 200mm x 100mm x 100mm or less.

The housing 12 may suitably be injection moulded in two parts from thermoplastic resin such as polyester. It is flame retardant and EMI shielded, resistant to hospital cleaners and disinfectants and UV. The monitor 10 is also shockproof, typically to IEC 60068-2-27, EN 1789, and/or IEC60601-1. The monitor 10 may also be shielded to electromagnetic interference (EMI) and ultraviolet (UV) radiation.

The monitor 10 is capable of operating at temperatures of -30 to 50°C and relative humidity of 15 to 95% (non-condensing). Monitor weight with battery is approximately 800 grams, and the charger, accessories and cables an additional 400 grams.

The screen 14 (an example of a user display and a user input device) is 5" diagonal, 800 x 400 pixels, and is touch operable with latex gloves. A loudspeaker 15, typically of 2W power, is included. A microphone 15a is also provided. The monitor 10 may be operable to use the microphone 15a to communicate with the remote web application 46 (voice through IP communication).

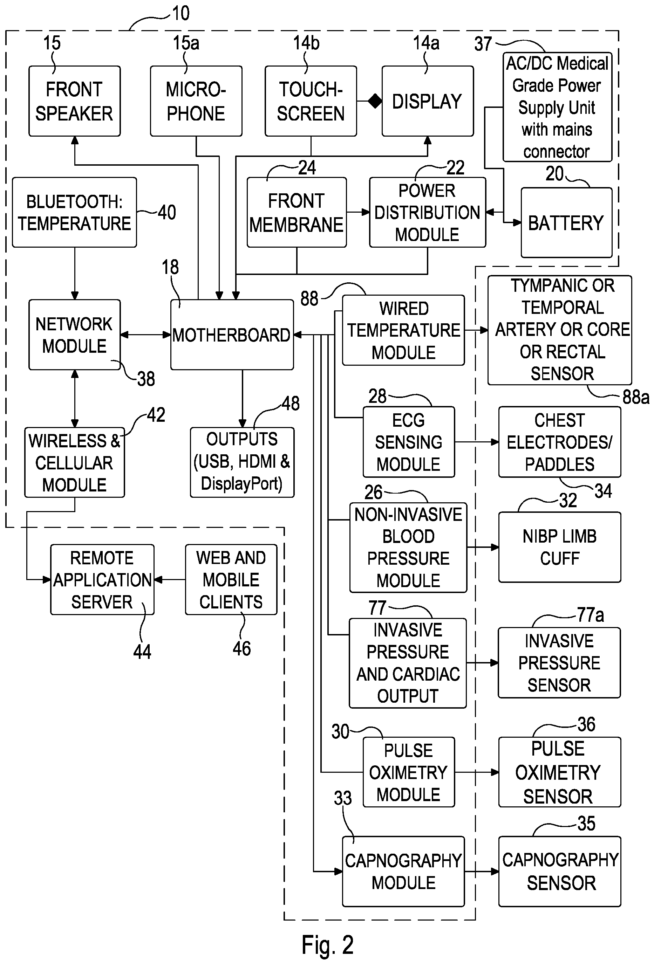

Fig. 2 shows in schematic block form the electronics within the monitor 10, and associated external parts. The monitor 10 is based on a motherboard 18 which may for example be an i.MX6Q Freescale microprocessor. The touch screen 14 comprises a display 14a and a touch sensitive layer 14b. The monitor 10 is powered by an internal battery 20 (an example of a power supply) via a power distribution module 22. The internal battery is located within the housing 12. A front membrane 24 (an example of a user display) provides one or more membrane switches to control power on/off and optionally other functions.

The battery 20 is most suitably a medical grade lithium ion battery, and is charged via mini-USB from an external power supply 37 when required; any suitable power supply 37 may be used, such as AC-DC regulated switch mode for use in a wall socket, or a medical grade power supply. Battery capacity may be sufficient for at least 48 hours in stand-by mode and 6 hours of continuous monitoring of ECG, SpO2, CO2 and two channels of temperature, with NIBP monitoring every 15 minutes.

The monitor 10 includes a number of sensor modules (an example of a measurement module) which cooperate with external sensors. The measurement modules are located in the housing 12. In this embodiment, these comprise a blood pressure module 26 (which may include invasive and non-invasive blood pressure), an ECG sensing module 28, a pulse oximetry module 30 and a capnography module 33. The monitor 10 may also include a cardiac output module 77 (invasive pressure and cardiac output). The cardiac output module may include a cardiac sensor 77a (invasive pressure sensor).

The blood pressure module 26 operates with a non-invasive blood pressure sensor 32 (or an invasive blood pressure sensor 32) in an arm or leg cuff. Suitable NIBP sensors and invasive pressure sensors are well known. The monitor 10 can be programmed to measure blood pressure at intervals of 10, 15, 30 or 60 minutes, with a manual start/stop function. Typical measurement time is 30 to 45 seconds (on deflation) and 15 to 30 seconds (on inflation), and typical measurement range systolic 20 to 260 mmHg, diastolic 10 to 220 mmHg. In a preferred embodiment, the blood pressure module 26 is a Sun Tech Medical blood pressure module. The blood pressure sensor 32 may be connected to connection port 16a.

The ECG sensing module 28 is connected in use to chest electrodes or paddles 34 of conventional type. In preferred embodiments, the monitor 10 is also usable for cardiac pacing. The chest electrodes or paddles 34 may be connected to connection port 16b.

The ECG is able to produce 3-, 5- and 12-lead ECG that will be input from 3, 5, 10 cables and sensing sources. The heart rate range is 30-300 bpm.

Pacing is external transcutaneous with rectilinear, constant current pulses with a pulse width of 40ms ±2ms and a pacer rate of 30 to 180bpm. Output current is typically 0 to 140mA.

The ECG electrodes 34 and module 28 can also be used to provide impedance pneumography. The displayed data may be both numeric breath rate and impedance waveform. The displayed breath rate is most suitably the average of the last ten breath-to-breath rates. Alarms may be set for high and low rates and no breath.

The pulse oximetry module 30 is connected in use to a pulse oximetry sensor 36 which will typically be a non-invasive light transmission sensor using LEDs and applied to a body part such as a finger-tip or ear lobe. In a preferred embodiment, the pulse oximetry module 30 is a Masimo MX5 module, which may optionally be used with Masimo multiple wavelength LED sensors, as the pulse oximetry sensor 36, to provide additional measurements, including Total Haemoglobin, Oxygen Content, Carboxyhaemoglobin, Methaemoglobin, and Pleth Variability Index in addition to pulse oximetry measurements of SpO2, pulse rate and perfusion index. The pulse oximetry sensor 36 may be connected to connection port 16c.

The capnography module 33 is connected to a capnography sensor 35 which will typically be a non-invasive and be located in a patient's airway. In a preferred embodiment, the capnography module 33 is a Covidien Microstream CO2 nano-mediCO2 module, which may optionally be used with a Covidien etCO2 sensor 35. The capnography module 33 may be operable to additionally measure other respiratory values, such as respiration rate, Integrated Pulmonary Index, SARA and Smart BDA, Apnoea Sat Alert and Oxygen desaturation index. The capnography module 33 may provide early indication of evolving respiratory compromise. The capnography sensor 35 may be connected to connection port 16d.

Each measurement module may be operable to provide instantaneous data measurements and/or historical data measurements. The data measurements may be presented numerically and/or visually in a plurality of different formats. Each measurement module may perform calculations that may be used as base sensed parameters, early deterioration notifications for individual parameters and different versions of patient early warning score based on multiple parameters. Each measurement module may be operable to predict trends and/or deterioration and provide warning alarms.

The monitor 10 also includes a tracking module. The monitor 10 alerts through a sound when the measurement modules are too far away/moved. When the measurement modules are out of Bluetooth range (up to 50 metres/160 feet in free open space only, in closed rooms this may be shorter) it will alert on the mono monitor or monitoring unit 10. The movement alert is triggered as soon as the measurement module is moved. (This feature pops up only when the monitoring unit 10 and Temp sensor range alert is enabled first, i.e. before the measurement module goes out of range from the monitoring unit.). The monitor 10 may be operable to provide a user with information relating to a last known location of the measurement module/sensor module. This information may be presented visually. The tracking module is operable to track the measurement modules in real time and present their location on a map, or the like.

The monitor 10 also includes a network module 38 controlling wireless communication with external devices. In this embodiment, temperature information is supplied from a temperature module 88 (wired temperature module) and temperature sensor 88a (tympanic or temporal artery or core or rectal sensor) via a Bluetooth module 40. The temperature sensor will typically be a non-contact infrared thermometer, many examples of which are well known.

The network module 38 also controls communication via a wireless and cellular module 42 with external devices or networks; this may be by cellular (mobile) telephony, or by Wi-Fi over a local area network, for example. In Fig. 2, the monitor 10 communicates via the wireless and cellular module 42 with a remote application server 44 which also communicates with other web and mobile clients 46. The other web and mobile clients 46 may include other similar vital signs monitors. Note that in the embodiment illustrated and described here the network module 38 is separate from the motherboard 18. However, it should be appreciated that the network module 38 may be within, or part, of the motherboard 38.

The monitor 10 has sufficient memory to retain data collected for a number of patients (typically up to 40). Patient ID can be entered via the touch screen. A typical memory capacity gives over 48 hours of trends at one minute intervals, 2000 time stamped events, and 64 monitor snapshots (max 20 seconds duration). The memory may be provided by an integrated micro SD card, or other such device.

Finally, the monitor 10 of Fig. 2 is provided with one or more outputs 48 such as USB, HDMI and DisplayPort. A preferred output arrangement is 2 x USB 3.0, HDMI, Ethernet, Wi-Fi and 4G.

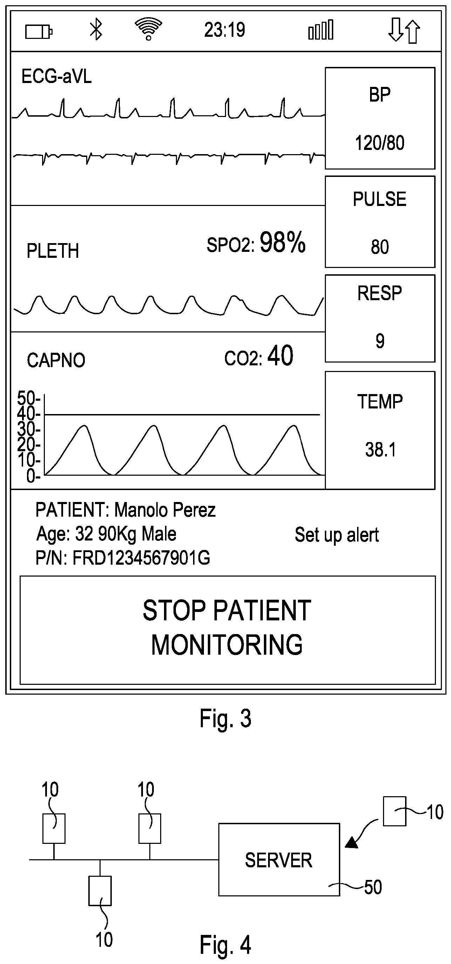

Fig. 3 shows an example of a screen display during monitoring. As will be seen, selected vital signs can be displayed as current readings, graphically, or both. The monitor and web application allow access of detailed views of each monitored parameter, which allows access to all calculations, measurement history and trends. The screen on the monitor and web application can be configured to select which parameter(s) is/are displayed.

The monitor as described can be used as a standalone unit in a healthcare setting such as a hospital or clinic, but is also suitable for use in the field, for example by ambulance or emergency medical technicians, or by medical staff in remote locations without sophisticated facilities. In addition, the monitor may also be used as part of a networked system.

The monitor 10 may also be provided with an ECG module capable of supplying cardiac pacing pulses.

Fig. 4 shows a networked system, typically in a hospital. A number of monitors 10, which may for example be in different wards or departments, communicate with a central server 50. Communication can be by any of the means discussed above, but will typically be by Ethernet or Wi-Fi. The central server can be used to integrate the monitored data with a patient record system, or to permit the patient readings to be shared in real time. It should be noted that the individual monitors 10 and the central server 50 are not functionally interdependent, and thus data can be sent from the monitor 10 asynchronously.

It is also possible to use a monitor 10 in the field and stream the data to a remote server, for example to enable a patient in an ambulance to be monitored by a hospital doctor.

The central server may also be used to aggregate data received from a number of monitors for example to conduct epidemiological studies. The availability of readily usable handheld monitors makes it possible to acquire large amounts of data for this purpose.

The remote application server may be able to use data to create a reporting framework. The reporting framework may be configured to store data that corresponds to vital sign monitoring sessions. The data may be presented using standard export formats. The formats may be: comma separated value (csv), MS Excel (xlsx), and PDF. Comma separated value (csv) formats allow for the manipulation of data through commercial off the shelf software. PDF formats enables the data to be presented using proprietary formats that may match the look and feel of a healthcare institution.

The reporting framework may be configured to run in its own web application. The reporting framework may be an independent module to achieve reusability.

The remote application may fully integrate the reporting framework with other patient file systems. This may decrease the navigation steps to get to patient information.

The monitor may also be operable to produce video reporting and data snapshots generation. The monitor may be operable to perform a feasibility study on how useful it would be to make the monitoring data anonymous so that it can be accessed openly throughout the world, most specifically by research centres and universities.

The monitor may have artificial intelligence (AI) algorithms that learn when data is normally requested. This provides the ability to automatically generate data reports based on the patient's condition or the context the patient is in.

The monitor according to the present invention may include the external sensors 32, 34, 35, 36.

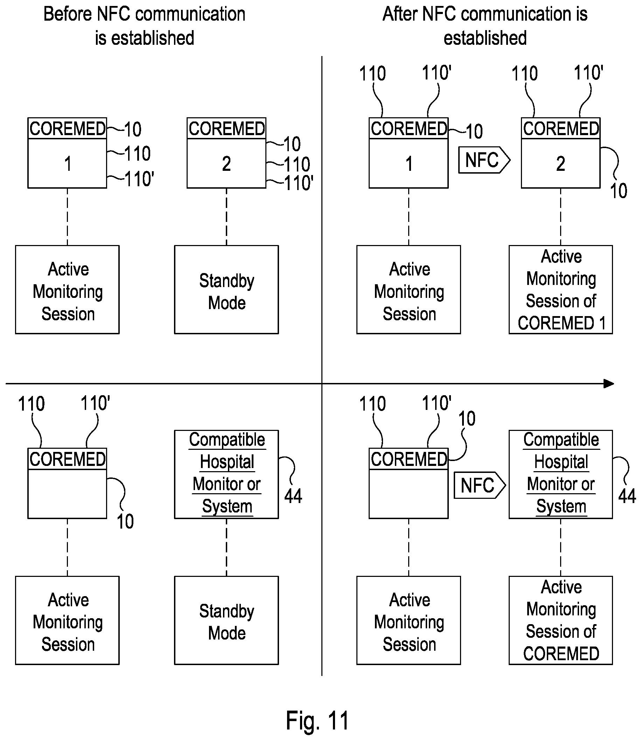

With reference to Fig. 11, the monitor 10 is configured to communicate with near field communication (NFC) technology. The monitor 10 has NFC transmission and receiving capabilities. The monitor 10 is configured to communicate with other monitors 10 or a remote application server 44, such as a compatible hospital monitor or system. When the NFC capability is activated on the monitor 10, the monitor 10 can communicate with other monitors 10 or remote application servers 44 that are compatible with the monitor 10 in locations such as hospitals through NFC technology. This feature may be used by the monitor 10 to "call" a recent monitoring session from the remote server 44 or the transmitting monitor 10 to the other monitor 10 or compatible station. This session will persist in the "new device" when NFC connectivity is lost and may be continued provided the new device has similar sensing capabilities.

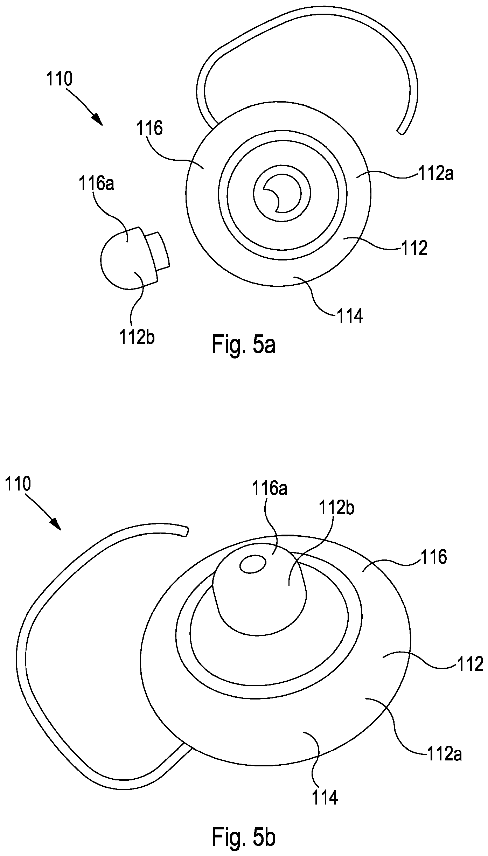

Referring to Figs. 5a and 5b, an example vital signs measurement apparatus 110 is illustrated which is suitable for use with the present invention. The apparatus 110 in includes a housing 112, a power supply 114 and a measurement module 116. As described further below, the measurement module 116 is operable to measure and monitor at least one respective vital sign. In the example illustrated and described here, the measurement module 116 is operable to measure the temperature of a patient 1. However, it should be appreciated that the measurement module 116 may be operable to measure and/or monitor vital signs selected from: pulse, blood pressure, invasive blood pressure, temperature, tympanic temperature, electrocardiogram (ECG), respiration, pulse oximetry, cardiac output and capnography.

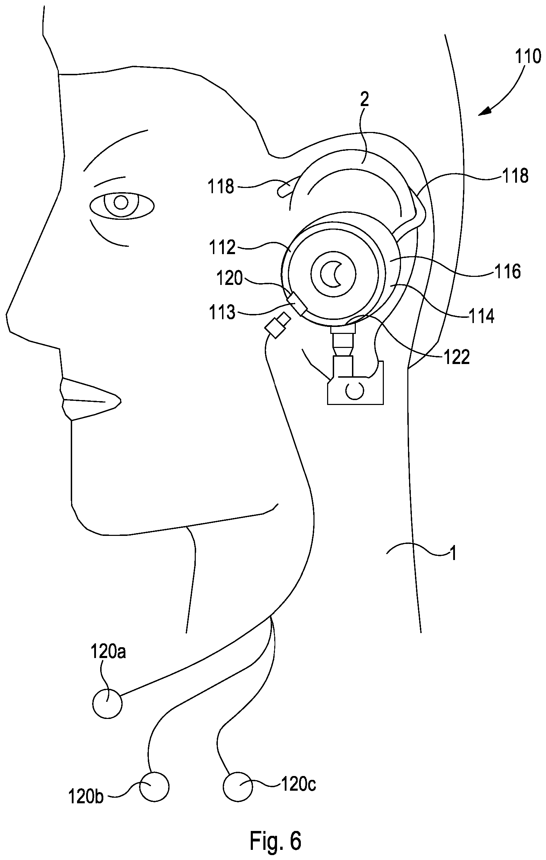

The apparatus 110 is designed to be wearable by the patient 1, as illustrated in Fig. 6. In the example (which is suitable for use with the invention) illustrated and described here, the apparatus 110 is a wearable in-ear electronic diagnostics device. The power supply 114 is located in the housing 112. As described further below, the measurement module 116 is mostly located in the housing 112. The housing 112 may be splash proof and dust resistant and may be made from a plastics material, polymer material or rubber material.

The power supply 114 may be a self-contained power supply, such as a rechargeable lithium-ion battery. Alternatively, the power supply 114 may be a mains power supply. The mains power supply may be a medical grade power supply.

In the example illustrated and described here, the measurement module 116 is an infrared sensor 116a that is operable to measure the temperature and/or pulse (heart rate) of the patient 1, or wearer of the apparatus 110. The infrared sensor 116a is arranged to measure the tympanic temperature of the patient 1, or wearer of the apparatus 110. The apparatus 110 thus has a tympanic temperature sensor.

As illustrated in Fig. 6, the housing 112 is attachable to the patient 1, or wearer of the apparatus 110. In the example illustrated and described here, the housing 112 includes an attachment member 118 that is used to attach the apparatus 110 to the ear 2 the patient 1. The attachment member 118 is hook member that is used to hook the apparatus 110 around the ear 2 of the patient 1. The attachment member 118 is a deformable wire that can be configured and adjusted to suit any patient 1. The attachment member 118 may be detachable from the housing 112. This allows the attachment member 118 to be disposable, if required. It should be appreciated that any suitable attachment member could be used to attach the apparatus 110 to the patient 1.

As illustrated in Figs. 5a, 5b and 6, the housing 112 is shaped such that the housing 112 may be fitted to the ear 2 of a patient, or wearer of the apparatus 110. In the example illustrated and described here, at least a portion of the housing 112 is shaped such that the at least a portion of the housing 112 may be fitted within an ear canal of the patient 1.

With reference to Figs. 5a and 5b, the housing 112 has a first portion 112a and a second portion 112b. The first portion 112a includes the power supply 114 and the measurement module 116. The second portion 112b of the housing 110 is shaped to allow part of the housing 112 to be fitted to the ear 2 of the patient 1. As illustrated in Figs. 5a and 5b, the second portion 112b is generally spherical in shape and is made from a resilient rubber material. This allows the second portion 112b of the housing 110 to be at least partially inserted into the ear canal of the patient 1. This holds the apparatus 110 in place relative to the patient's ear 2. The second portion 112b of the housing also arranged such that the radiation emitted from the infrared sensor 116a is directed towards the tympanum of the patient 1.

The infrared sensor 116a may be located in the first portion 112a of the housing 112 or the second portion 112b of the housing 112.

The second portion 112b of the housing 112 may be detachable from the housing 112. This allows the second portion 112b to be disposable, if required.

The apparatus 110 may further comprise one or more additional measurement modules. The additional measurement modules may be operable to measure and/or monitor vital signs selected from: pulse, blood pressure, invasive blood pressure, temperature, tympanic temperature, electrocardiogram (ECG), respiration, pulse oximetry, cardiac output and capnography.

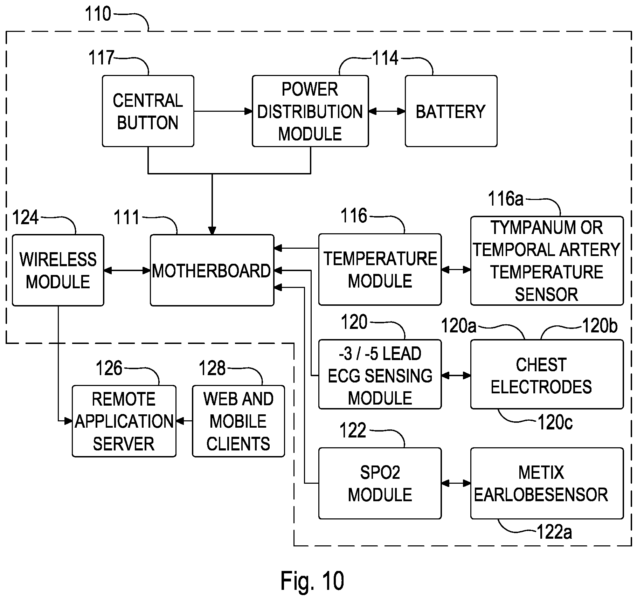

As illustrated in Figs. 6 and 10, the apparatus 110 may include one or more additional measurement modules 116. In the embodiment illustrated and described here, the apparatus 110 includes an additional electrocardiogram (ECG) measurement module 120 and an additional pulse oximetry measurement module 122.

The ECG measurement module 120 is removably attachable to the housing 112, as illustrated in Fig. 6 at connection point 113. The ECG measurement module 120 include sensors 120a, 120b and 120c that are attachable to the patient 1. The sensors 120a, 120b and 120c may be chest electrodes, paddles, or the like. The ECG module 120 may be operable with 3, 5 or 12 lead cables. The sensors 120a, 120b and 120c may be disposable. The ECG measurement module 120 may be operable to measure heart rate in the range 30 to 300 bpm. The ECG measurement module 120 may be operable to measure cardiac pacing. The ECG measurement module 120 may be operable to measure rectilinear, constant width current pulses of 40 ms ± 2 ms at a pacer rate of 30 to 180 bpm. Such measurements may be external transcutaneous.

The ECG measurement module 120 may be operable to provide impedance pneumography. The ECG measurement module 120 may be operable to measure breath rate. The ECG measurement module 120 may be operable to measure breath rate between 2 to 150 breaths per minute. The ECG measurement module 20 may be operable to display the numeric breath rate. The ECG measurement module 120 may be operable to display the impedance waveform. The ECG measurement module 120 may be operable to measure and/or monitor an averaged breath rate. The ECG measurement module 120 may be operable to activate an alarm for low, high and no breath rates. The ECG measurement module 120 may be operable to activate arrhythmia detection.

The pulse oximetry measurement module 122 may be connected to an earlobe or nose of the patient 1.

The pulse oximetry measurement module 122 may be a non-invasive measurement module. The pulse oximetry measurement sensor 122a may be a non-invasive light transmission measurement module. The pulse oximetry measurement module 122 may be operable to measure SpO2, pulse rate and perfusion index. The pulse oximetry measurement module 122 may be operable to additionally measure pleth variability index.

The measurement module 116 (and additional measurement modules, if present) may be operable to provide instantaneous data measurements and/or historical data measurements. The data measurements may be presented numerically and/or visually in a plurality of different formats. The measurement module 116 may perform calculations that may be used as base sensed parameters, early deterioration notifications for individual parameters and different versions of patient early warning score based on multiple parameters. The measurement module 116 (and additional measurement modules, if present) may be operable to predict trends and/or deterioration and provide warning alarms. The measurement module 116 (and additional measurement modules, if present) may be operable to monitor and/or process the measured data.

The apparatus 110 may include one or more alarm devices (not illustrated), such that the apparatus 110 may be operable to signal an alarm upon measurement of a vital sign having one or more predetermined signals or predetermined values or conditions. The alarm may be made through a speaker device which emits an audible warning sound.

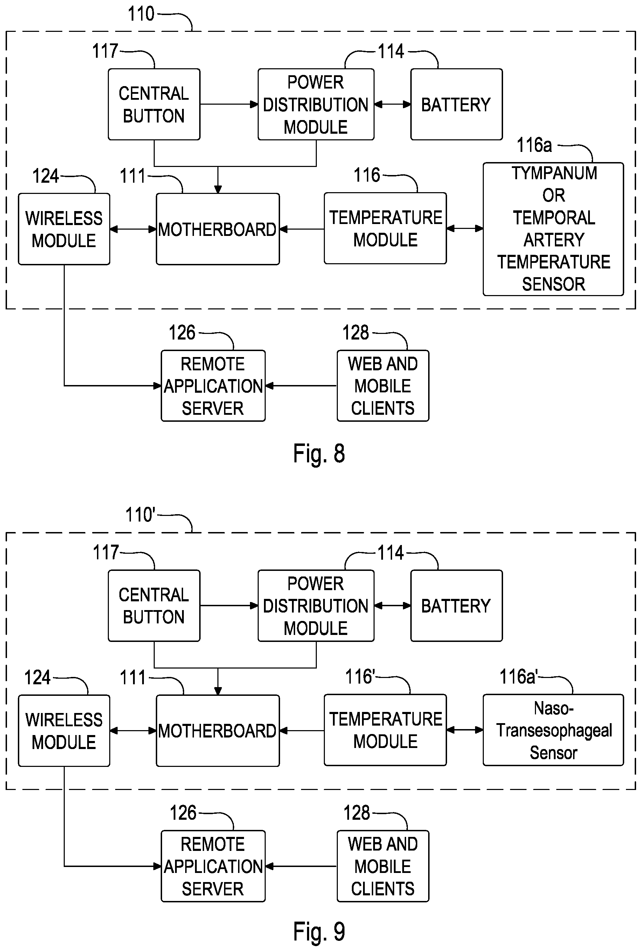

As illustrated in Fig. 8, the apparatus 110 is based on a motherboard 111. The measurement module 116 (and additional measurement modules, if present) is operable to communicate with one or more external devices. The external devices may be a mobile telecommunications device, such as a smart phone, tablet, or the like. The apparatus 110 includes an operating button 117 that communicates with the motherboard 111 and the power supply 114.

The measurement module 116 (and additional measurement modules, if present) communicates with one or more external devices using a network module 124. The network module 124 is a wireless network module. The apparatus 110, or the measurement module 116 (and additional measurement modules, if present), may therefore comprise a network module 124.

The network module 124 is operable to control the communication between the measurement module 116 (and additional measurement modules, if present) and one or more external devices or networks, such as a remote application server 126. The network module 124 is operable to wirelessly control the communication between the measurement module 116 (and additional measurement modules, if present) and one or more external devices or networks 126. The communication protocol may be Bluetooth, Bluetooth 4.0, Bluetooth 4.1, or the like. The communication may be by cellular (mobile) telephony, or by Wi-Fi over a local area network (LAN), or the like.

As described above, the apparatus 110 is configured to be operable with a remote application server 126. The remote application server 126 is also operable to communicate with other devices, web applications or mobile clients 128. The remote application server 126 may also be operable to communicate with other vital sign measurement apparatus 116, 120, 122 etc. The apparatus 110 is therefore operable with the remote application server 126 via the network module 124.

The apparatus 110 may also include a tracking module (not illustrated) that allows the apparatus 110 to be tracked. The tracking module may include a geolocation feature through the remote web application 126.

The apparatus 110 may also include a tracking module. The tracking module may be operable to detect when an external device to which the apparatus 110 is in communication with moves out of range with respect to the apparatus 110. The apparatus 110 alerts through a sound when the external device is too far away/moved. When out of Bluetooth range (up to 50 metres/160 feet in free open space only, in closed rooms this may be shorter) it will alert on the external device. The movement alert is triggered as soon as the apparatus 110 is moved. (This feature pops up only when the external device and apparatus range alert is enabled first, i.e. before the external device goes out of range from the apparatus 110.)

The apparatus 110 may be operable to control the operation and measurement of one or more different parameters or functions of the apparatus. This may be switching the measurement functions between ECG and SPO2, as an example.

The apparatus 110 also has an internal memory capacity that may be used to store firmware, or the like. This memory capacity may, as an example, be 264kb.

In use the apparatus 110 provides a readily-portable, self-contained, partially disposable device which can monitor vital signs not only in sophisticated hospitals but also in the field, for example in ambulances and in remote unsophisticated medical facilities.

The apparatus 110 as described can be used as a standalone unit in a healthcare setting such as a hospital or clinic, but is also suitable for use in the field, for example by ambulance or emergency medical technicians, or by medical staff in remote locations without sophisticated facilities. In addition, the apparatus 110 may also be used as part of a networked system.

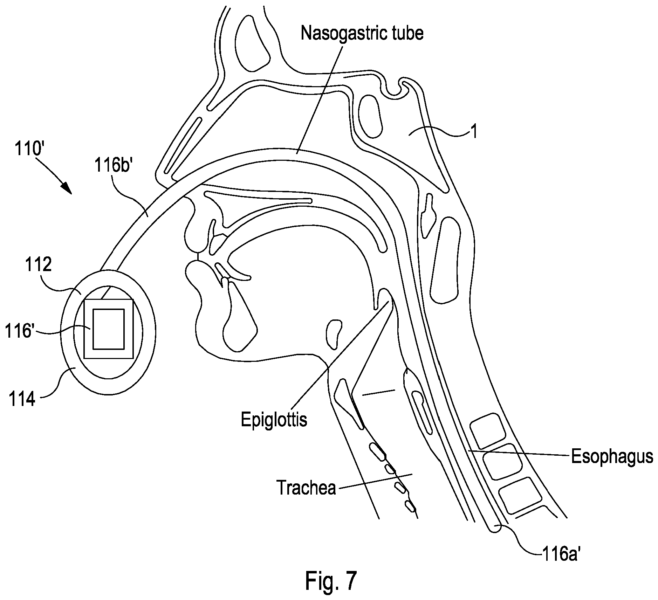

Figs. 7 and 9 illustrate a second example of vital signs measurement apparatus 110', which can be used with the present invention. The difference between the vital signs measurement apparatus 110 of Figs. 5a, 5b, 6, 8 and 10 and the vital signs measurement apparatus 110' of Figs. 7 and 9 is that the vital signs measurement apparatus 110' of Figs. 7 and 9 is a transoesophageal diagnostics device that measures the core temperature of the patient 1. The housing 112, power supply 114 etc. are generally the same between the examples. The housing 112 of the apparatus 110' is not configured to be fitted to the ear 2 of the patient 1.

The apparatus 110' is designed to be wearable by the patient 1. The apparatus 110' may be removably attachable to the patient 1. This may be by a releasable fastening device, such as a clip etc.

In the example illustrated and described here, the measurement module 116' is an infrared sensor 116a' that is operable to measure the temperature and/or pulse (heart rate) of the patient 1, or wearer of the apparatus 110'. The infrared sensor 116a' is arranged to measure the core temperature of the patient 1, or wearer of the apparatus 110'. The apparatus 110' thus has a core temperature sensor. The infrared sensor 116a' is a naso-transoesophageal sensor.

The temperature sensor 116a' is located within a tube member 116b' (an example of an external member), the tube member 116b' is removably attachable to the housing 112. The tube member 116b' is configurable to be insertable into the oesophagus of the patient 1, as illustrated in Fig. 7. The tube member 116b' may be a nasogastric tube. The tube member 116b' may be made from a polymer material. The tube member 116b' may be configured such that it locatable within the oesophagus of the patient 1. The tube member 116b' may be configured such that it remains within the oesophagus of the patient 1 during use without entering the gastric cavity of the patient 1. The temperature sensor 116a' may be located towards the end of the tube 116b' that is remote from the housing 112. The tube member 116b' may be a sealed tube member. The tube member 116b' may be sealed at the end of the tube 116b' that is remote from the housing 112. The measurement module 116' may include a plurality of core temperature sensors 116a'. Each core temperature sensor 116a' may be located within the tube member 116b'.

Again, with reference to Fig. 11, the apparatus 110, 110' is configured to communicate with near field communication (NFC) technology. The apparatus 110, 110' has NFC transmission and receiving capabilities. The apparatus 110 is configured to communicate with other apparatuses 110, 110' or a remote application server 44, such as a compatible hospital monitor or system. When the NFC capability is activated on the apparatus 110, 110', the apparatus 110, 110' can communicate with other apparatuses 110, 110' or remote application servers 44 that are compatible with the apparatus 110, 110' in locations such as hospitals through NFC technology. This feature may be used by the apparatus 110, 110' to "call" a recent monitoring session from the remote server 44 or the transmitting apparatus 110, 110' to the other apparatus 110, 110' or compatible station. This session will persist in the "new device" when NFC connectivity is lost and may be continued provided the new device has similar sensing capabilities.
The operation and benefits of the vital signs measurement apparatus 110' are the same as the operation and benefits of the vital signs measurement apparatus 110.
The present invention thus provides a vital signs monitor which is readily portable, self-contained, and easy to use.

Modifications and improvements may be made to the above without departing from the scope of the present invention, which is defined by the appended claims.
For example, it should be appreciated that the apparatus may include one or more vital signs measurement modules, as described above. Each vital signs measurement module may be operable to measure and monitor a different vital sign.
It should also be appreciated that each measurement module may include one or more sensors. These sensors may be infrared sensors.
Also, it should be appreciated that the infrared sensors may be used to measure both temperature (tympanic or core) and pulse rate.

## Claims

1. A vital signs monitor (10) comprising:
a housing (12);
a power supply (20);
a plurality of measurement modules within the housing (12), each module being operable to measure and monitor at least one respective vital sign;
one or more inputs (16) for connection to sensors applied to a patient and arranged to supply sensor signals to respective measurement modules; and
a user display (14a), wherein the measurement modules are operable to communicate wirelessly with external sensors (32, 34, 35, 36), the vital signs monitor (10) has near field communication (NFC) transmission and receiving capabilities, such that the monitor (10) may communicate with a further vital signs monitor (10) using NFC technology, the further vital signs monitor (10) comprising:
a housing (12);
a power supply (20);
a plurality of measurement modules within the housing (12), each module being operable to measure and monitor at least one respective vital sign;
one or more inputs (16) for connection to sensors applied to a patient and arranged to supply sensor signals to respective measurement modules; and
a user display (14a), and
**characterised in that** the vital signs monitor (10) includes one or more tracking devices, the one or more tracking devices being operable to signal an alarm when an external sensor (32, 34, 35, 36) moves out of a predetermined range with respect to the vital signs monitor (10).

2. A vital signs monitoring system comprising:
two or more vital signs monitors (10) of claim 1; and
a server (44),
wherein each vital signs monitor (10) is independently operable to provide a local display (14a), and data is transferred between each vital signs monitor (10) and the server (44), and
wherein each vital signs monitor (10) may communicate with the other vital signs monitor(s) (10) using NFC technology.

3. A vital signs monitoring system according to claim 2, wherein the data is transferred between each vital signs monitor (10) and the server (44) asynchronously.

4. A vital signs monitoring system according to claim 2 or claim 3, wherein the data is stored in both the respective vital signs monitor (10) and the server (44).

5. A vital signs monitoring system according to any one of claims 2 to 4, wherein the server (44) is operable to aggregate data from a population of individual data sets.

6. A vital signs monitor (10) according to claim 1, wherein the vital signs monitor (10) is a hand-held device.

7. A vital signs monitor (10) according to claim 1 or claim 6, wherein each measurement module is operable to provide instantaneous data measurements and/or historical data measurements.

8. A vital signs monitor (10) according to claim 7, wherein each measurement module is operable to predict trends and/or deterioration and provide warning alarms to a user.

9. A vital signs monitor (10) according to any one of claims 1 or 6 to 8, wherein the monitor (10) includes one or more alarm devices, the one or more alarm devices being operable to signal an alarm upon measurement of a vital sign having one or more predetermined signals or predetermined values or conditions.

10. A vital signs monitor (10) according to any one of claims 1 or 6 to 9, wherein the monitor (10) also comprises a network module (38), the network module (38) being operable to control the communication between the measurement module sensors and the measurement modules.

11. A vital signs monitor (10) according to claim 10, wherein the network module (38) is operable to wirelessly control the communication between the measurement module sensors and the measurement modules.

12. A vital signs monitor (10) according to claim 10 or claim 11, wherein the network module (38) is also operable to control the communication between the vital signs monitor (10) and/or the measurement modules thereof, with one or more external devices or networks.

13. A vital signs monitor (10) according to claim 12, wherein the network module (38) is operable to wirelessly control the communication between the vital signs monitor (10) and/or the measurement modules thereof, with one or more external devices or networks.

14. A vital signs monitor (10) according to any one of claims 1 or 6 to 13, wherein the vital signs monitor (10) is configured to be operable with a remote application server (44), the remote application server (44) being operable to communicate with other devices, web applications, mobile clients, or other vital signs monitors (10).

15. A vital signs monitor (10) according to any one of claims 1 or 6 to 14, wherein the vital signs monitor (10) further comprises an output device for communication with a remote server (44).

## Patentansprüche

1. Patientenmonitor (10), Folgendes
umfassend: ein Gehäuse (12);
ein Netzanschlussgerät (20);
mehrere Messmodule im Gehäuse (12), wobei jedes Modul zur Messung und Überwachung mindestens eines jeweiligen Vitalzeichens ausgebildet ist;
einen oder mehrere Eingänge (16) zur Verbindung mit an einem Patienten angelegten Sensoren, die so angeordnet sind, dass Sensorsignale an entsprechende Messmodule geleitet werden; und
eine Benutzeranzeige (14a), wobei die Messmodule zur drahtlosen Kommunikation mit externen Sensoren (32, 34, 35, 36) ausgebildet sind, der Patientenmonitor (10) Sende- und Empfangsfähigkeiten in der Nahfeldkommunikation (NFC) aufweist, so dass der Monitor (10) mit einem weiteren Patientenmonitor (10) unter Verwendung der NFC-Technik kommunizieren kann, wobei der weitere Patientenmonitor (10) Folgendes umfasst:
ein Gehäuse (12);
ein Netzanschlussgerät (20);
mehrere Messmodule im Gehäuse (12), wobei jedes Modul zur Messung und Überwachung mindestens eines jeweiligen Vitalzeichens ausgebildet ist;
einen oder mehrere Eingänge (16) zur Verbindung mit an einem Patienten angelegten Sensoren, die so angeordnet sind, dass Sensorsignale an entsprechende Messmodule geleitet werden; und
eine Benutzeranzeige (14a) und
**dadurch gekennzeichnet, dass** der Patientenmonitor (10) eine oder mehrere Nachverfolgungsvorrichtungen umfasst, wobei die eine oder die mehreren Nachverfolgungsvorrichtungen so ausgebildet sind, dass sie einen Alarm anzeigen, wenn ein externer Sensor (32, 34, 35, 36) einen vorbestimmten Bereich in Bezug auf den Patientenmonitor (10) verlässt.

2. Anlage zur Überwachung der Vitalfunktionen, Folgendes umfassend:
zwei oder mehrere Patientenmonitore (10) nach
Anspruch 1; und einen Server (44),
wobei jeder Patientenmonitor (10) unabhängig zur Bereitstellung einer lokalen Anzeige (14a) ausgebildet ist und Daten zwischen den jeweiligen Patientenmonitoren (10) und dem Server (44) übertragen werden und wobei jeder Patientenmonitor (10) mit dem/den anderen Patientenmonitor(en) (10) unter Verwendung der NFC-Technologie kommunizieren kann.

3. Anlage zur Überwachung der Vitalfunktionen nach Anspruch 2, wobei die Daten zwischen den jeweiligen Patientenmonitoren (10) und dem Server (44) asynchron übertragen werden.

4. Anlage zur Überwachung der Vitalfunktionen nach Anspruch 2 oder 3, wobei die Daten sowohl im jeweiligen Patientenmonitor (10) als auch im Server (44) gespeichert werden.

5. Anlage zur Überwachung der Vitalfunktionen nach einem der Ansprüche 2 bis 4, wobei der Server (44) so ausgebildet ist, dass er Daten aus einer Population individueller Datensätze aggregiert.

6. Patientenmonitor (10) nach Anspruch 1, wobei der Patientenmonitor (10) ein Handgerät ist.

7. Patientenmonitor (10) nach Anspruch 1 oder Anspruch 6, wobei jedes Messmodul zur Bereitstellung aktueller Messdaten bzw. Verlaufsmessdaten ausgebildet ist.

8. Patientenmonitor (10) nach Anspruch 7, wobei jedes Messmodul so ausgebildet ist, dass es Trends bzw. Verschlechterungen vorhersagen und Warnmeldungen an einen Benutzer ausgeben kann.

9. Patientenmonitor (10) nach einem der Ansprüche 1 oder 6 bis 8, wobei der Monitor (10) eine oder mehrere Alarmvorrichtungen enthält, wobei die eine oder die mehreren Alarmvorrichtungen so ausgebildet sind, dass sie bei der Messung einer Vitalfunktion mit einem oder mehreren vorgegebenen Signalen oder vorgegebenen Werten oder Bedingungen einen Alarm ausgeben.

10. Patientenmonitor (10) nach einem der Ansprüche 1 oder 6 bis 9, wobei der Monitor (10) auch ein Netzwerkmodul (38) umfasst, wobei das Netzwerkmodul (38) zur Steuerung der Kommunikation zwischen den Messmodulsensoren und den Messmodulen ausgebildet ist.

11. Patientenmonitor (10) nach Anspruch 10, wobei das Netzwerkmodul (38) zur drahtlosen Steuerung der Kommunikation zwischen den Messmodulsensoren und den Messmodulen ausgebildet ist.

12. Patientenmonitor (10) nach Anspruch 10 oder 11, wobei das Netzwerkmodul (38) auch zur Steuerung der Kommunikation zwischen dem Patientenmonitor (10) bzw. dessen Messmodulen mit einem oder mehreren externen Geräten oder Netzwerken ausgebildet ist.

13. Patientenmonitor (10) nach Anspruch 12, wobei das Netzwerkmodul (38) zur drahtlosen Steuerung der Kommunikation zwischen dem Patientenmonitor (10) bzw. dessen Messmodulen mit einem oder mehreren externen Geräten oder Netzwerken ausgebildet ist.

14. Patientenmonitor (10) gemäß einem der Ansprüche 1 oder 6 bis 13, wobei der Patientenmonitor (10) so konfiguriert ist, dass er mit einem entfernten Anwendungsserver (44) betrieben werden kann, wobei der entfernte Anwendungsserver (44) so betrieben werden kann, dass er mit anderen Geräten, Web-Apps, mobilen Clients oder anderen Patientenmonitoren (10) kommunizieren kann.

15. Patientenmonitor (10) gemäß einem der Ansprüche 1 oder 6 bis 14, wobei der Patientenmonitor (10) ferner eine Ausgabevorrichtung zur Kommunikation mit einem entfernten Server (44) umfasst.

## Revendications

1. Moniteur de signes vitaux (10)
comprenant : un boîtier (12) ;
une alimentation électrique (20) ;
une pluralité de modules de mesure dans le boîtier (12), chaque module pouvant être utilisé pour mesurer et surveiller au moins un signe vital respectif ;
une ou plusieurs entrées (16) pour la connexion à des capteurs appliqués à un patient et agencées pour fournir des signaux de capteur à des modules de mesure respectifs ; et
un affichage utilisateur (14a), dans lequel les modules de mesure peuvent fonctionner pour communiquer sans fil avec des capteurs externes (32, 34, 35, 36), le moniteur de signes vitaux (10) a des capacités de transmission et de réception de communication en champ proche (NFC), de sorte que le moniteur (10) peut communiquer avec un autre moniteur de signes vitaux (10) en utilisant la technologie NFC, l'autre moniteur de signes vitaux (10) comprenant :
un boîtier (12) ;
une alimentation électrique (20) ;
une pluralité de modules de mesure dans le boîtier (12), chaque module pouvant être utilisé pour mesurer et surveiller au moins un signe vital respectif ;
une ou plusieurs entrées (16) pour la connexion à des capteurs appliqués à un patient et agencées pour fournir des signaux de capteur à des modules de mesure respectifs ; et
un affichage utilisateur (14a), et
**caractérisé en ce que** le moniteur de signes vitaux (10) comprend un ou plusieurs dispositifs de suivi, le ou les dispositifs de suivi pouvant fonctionner pour signaler une alarme lorsqu'un capteur externe (32, 34, 35, 36) se déplace hors d'une plage prédéterminée par rapport au moniteur de signes vitaux (10).

2. Système de surveillance des signes vitaux comprenant :
deux moniteurs de signes vitaux ou plus (10) selon
la revendication 1 ; et un serveur (44),
dans lequel chaque moniteur de signes vitaux (10) peut fonctionner indépendamment pour fournir un affichage local (14a), et des données sont transférées entre chaque moniteur de signes vitaux (10) et le serveur (44), et
dans lequel chaque moniteur de signes vitaux (10) peut communiquer avec le ou les autres moniteurs de signes vitaux (10) en utilisant la technologie NFC.

3. Système de surveillance de signes vitaux selon la revendication 2, dans lequel les données sont transférées entre chaque moniteur de signes vitaux (10) et le serveur (44) de manière asynchrone.

4. Système de surveillance de signes vitaux selon la revendication 2 ou la revendication 3, dans lequel les données sont stockées à la fois dans le moniteur de signes vitaux (10) respectif et dans le serveur (44).

5. Système de surveillance de signes vitaux selon l'une quelconque des revendications 2 à 4, dans lequel le serveur (44) est utilisable pour agréger des données provenant d'une population d'ensembles de données individuels.

6. Moniteur de signes vitaux (10) selon la revendication 1, dans lequel le moniteur de signes vitaux (10) est un appareil portatif.

7. Moniteur de signes vitaux (10) selon la revendication 1 ou la revendication 6, dans lequel chaque module de mesure est utilisable pour fournir des mesures de données instantanées et/ou des mesures de données historiques.

8. Moniteur de signes vitaux (10) selon la revendication 7, dans lequel chaque module de mesure est utilisable pour prédire des tendances et/ou une détérioration et fournir des alarmes d'avertissement à un utilisateur.

9. Moniteur de signes vitaux (10) selon l'une quelconque des revendications 1 ou 6 à 8, dans lequel le moniteur (10) comprend un ou plusieurs dispositifs d'alarme, le ou les dispositifs d'alarme pouvant être actionnés pour signaler une alarme lors de la mesure d'un signe vital ayant un ou plusieurs signaux prédéterminés ou des valeurs ou conditions prédéterminées.

10. Moniteur de signes vitaux (10) selon l'une quelconque des revendications 1 ou 6 à 9, dans lequel le moniteur (10) comprend également un module de réseau (38), le module de réseau (38) pouvant fonctionner pour commander la communication entre les capteurs du module de mesure et les modules de mesure.

11. Moniteur de signes vitaux (10) selon la revendication 10, dans lequel le module de réseau (38) peut fonctionner pour commander sans fil la communication entre les capteurs du module de mesure et les modules de mesure.

12. Moniteur de signes vitaux (10) selon la revendication 10 ou la revendication 11, dans lequel le module de réseau (38) est également utilisable pour commander la communication entre le moniteur de signes vitaux (10) et/ou ses modules de mesure, avec un ou plusieurs dispositifs ou réseaux externes.

13. Moniteur de signes vitaux (10) selon la revendication 12, dans lequel le module de réseau (38) est également utilisable pour commander la communication entre le moniteur de signes vitaux (10) et/ou ses modules de mesure, avec un ou plusieurs dispositifs ou réseaux externes.

14. Moniteur de signes vitaux (10) selon l'une quelconque des revendications 1 ou 6 à 13, dans lequel le moniteur de signes vitaux (10) est configuré pour pouvoir fonctionner avec un serveur d'application distant (44), le serveur d'application distant (44) pouvant fonctionner pour communiquer avec d'autres dispositifs, applications Web, clients mobiles ou autres moniteurs de signes vitaux (10).

15. Moniteur de signes vitaux (10) selon l'une quelconque des revendications 1 ou 6 à 14, dans lequel le moniteur de signes vitaux (10) comprend en outre un dispositif de sortie pour la communication avec un serveur distant (44).
